# EUROPEAN PATENT APPLICATION

(11) **EP 4 491 219 A1**
(43) Date of publication of application: **15.01.2025**
(21) Application number: 23876512.7
(22) Date of filing: 25.09.2023
(51) Int. Cl.: A61N 5/10

(54) **PLACEMENT PLATFORM POSITIONING SYSTEM, PLACEMENT PLATFORM POSITIONING METHOD, AND RADIATION THERAPY SYSTEM**

(30) Priority: 14.10.2022 CN 202211259602
(71) Applicant: Neuboron Therapy System Ltd., Xiamen, Fujian 361026 (CN)
(72) Inventor: YAN, Fazhi, Nanjing, Jiangsu 211112 (CN); GONG, Qiuping, Nanjing, Jiangsu 211112 (CN)
(74) Representative: Gunzelmann, Rainer
(86) International application number: PCT/CN2023/120922
(87) International publication number: WO 2024/078307

(57) **Abstract**

A placement platform (40) positioning system, which comprises: a placement platform (40), a placement platform positioning apparatus (60), a placement platform transport apparatus (401), and a connection apparatus (300). The placement platform (40) is used for bearing a patient (200). The placement platform positioning apparatus (60) is used for moving and positioning the placement platform (40). The placement platform transport apparatus (401) is used for supporting and moving the placement platform (40). And the connection apparatus (300) is used for locking the placement platform (40) to the placement platform positioning apparatus (60). Providing the connection apparatus (300) allows the placement platform positioning apparatus (60) to be quickly locked and secured to the placement platform (40), thereby reducing unnecessary positioning steps during treatment. A placement platform (40) positioning method, which comprises: rotating a first connection assembly (500) to a clamping position, such that the first connection assembly (500) and a second connection assembly (700) are locked; moving the placement platform positioning apparatus (60), and positioning the placement platform (40). A locking action between the placement platform positioning apparatus (60) and the placement platform (40) is completed in a single step and the operation is simple and convenient, thereby greatly reducing the overall positioning time of the placement platform (40).

## Description

### TECHNICAL FIELD

The present disclosure relates to a treatment positioning system and method, and in particular to a placement table positioning system, a placement table positioning method, and a radiotherapy system.

### BACKGROUND

With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors.

In order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively. With specific aggregation of boron-containing drugs in tumor cells, and in cooperation with accurate neutron beam irradiation, boron neutron capture therapy (BNCT) in neutron capture therapy serves as a better alternative to treat the cancers.

For the sake of the accurate neutron beam irradiation, in the simulated positioning room, the tumor site of the patient is to be accurately positioned and labeled through a computed tomography (CT) image and the like in combination with a laser positioning system. In the treatment room, the patient is to be positioned through the laser positioning system in combination with the marker labeled in the simulated positioning room, such that the neutron beam is aligned with the tumor for irradiation. The laser positioning system includes a laser emitter. The laser emitter is fixedly provided on a wall or a ceiling of the simulated positioning room and the treatment room. Hence, the positioning device is required to drive the placement table and position the placement table quickly and accurately. In the neutron capture therapy, how to lock the placement table and the positioning device quickly and accurately to achieve accurate positioning is a crucial step for the neutron beam to deliver accurate treatment.

Therefore, it is desired to provide a placement table positioning system and a placement table positioning method to solve the above problem.

### SUMMARY

In order to solve the problem on how to quickly lock a placement table and a positioning device in the conventional art to achieve an accurate positioning marker, a first aspect of the present disclosure provides a placement table positioning system, including a placement table, a placement table positioning device, a placement table transferring device, and a connecting device, where the placement table is configured to bear a patient; the placement table positioning device is configured to move and position the placement table; the placement table transferring device is configured to support and move the placement table; the connecting device has a clamping position where the placement table and the placement table positioning device are locked and a releasing position where the placement table and the placement table positioning device are separated; the connecting device includes a first connecting assembly and a second connecting assembly that cooperate with each other; and the first connecting assembly and the second connecting assembly are respectively oppositely provided on the placement table and the placement table positioning device. The placement table and the patient are transferred to the placement table positioning device through the placement table transferring device. The placement table and the placement table positioning device can be quickly clamped and locked through the connecting device to reduce the positioning time. With the connecting device in the simulated positioning room, the placement table can be quickly positioned in combination with the placement table positioning device to obtain an accurate positioning marker, thereby omitting working time of simulated positioning on the patient before irradiation in the treatment room. On the other hand, with the connecting device in the treatment room, the placement table and the patient can be positioned quickly and accurately in combination with the placement table positioning device according to the positioning marker obtained in the simulated positioning room, thereby effectively preventing the unnecessary particle irradiation dose on the patient and the medical staff in the treatment room.

In an embodiment, the first connecting assembly includes a mounting table and a locking member provided on the mounting table; a notch aligned and matched with the second connecting assembly is formed in the mounting table; and after rotating a preset angle, the locking member can be clamped with the second connecting assembly passing through the notch. The whole first connecting assembly is mounted through the mounting table. The locking member is provided on the mounting table. After the mounting table is aligned with the second connecting assembly, a portion of the second connecting assembly can pass through the notch in the mounting table, such that the first connecting assembly and the second connecting assembly are closely attached. By this time, rotating the locking member to the clamping position can realize locking. With the one-stop locking action and the simple operation, the time for positioning the whole placement table is greatly saved.

In an embodiment, the locking member includes a driving portion movably connected relative to the mounting table, a locking portion, and a limiting portion provided on the mounting table; the driving portion is configured to drive the locking portion to rotate to the clamping position where the locking portion is locked with the second connecting assembly or the releasing position where the locking portion is separated from the second connecting portion; and the limiting portion is provided with a limiting end for limiting the driving portion. The driving portion is driven manually to rotate. The driving portion in rotation drives the locking portion to rotate. The locking portion is initially away from the notch, without hindering alignment and attachment of the first connecting assembly and the second connecting assembly. The rotary locking process of the locking portion is a process of gradually getting close to notches at two sides of the locking portion, until the locking portion is clamped with the portion of the second connecting assembly passing through the notch. By this time, the locking member is locked with the second connecting assembly, such that the placement table positioning device and the placement table are locked, thereby facilitating subsequent movement and positioning of the placement table positioning device on the placement table. The limiting portion is configured to limit a rotating amplitude of the driving portion. When the driving portion rotates to a first limiting end of the limiting portion, the locking portion and the second connecting assembly are separated and unlocked, and the placement table positioning device can be separated from the placement table. When the driving portion rotates to a second limiting end of the limiting portion, the locking portion and the second connecting assembly are connected and locked, and the placement table positioning device can be locked with the placement table.

In an embodiment, a side of the limiting end close to the driving portion is provided with a first stop member; the driving portion is provided with a second stop member corresponding to the first stop member; and when the locking portion is located at the clamping position, the second stop member and the first stop member are connected to lock the driving portion and the locking portion. The first stop member and the second stop member are configured to fasten the driving portion. When the driving portion rotates to a position where the second stop member and the first stop member are connected, the driving portion is fastened to stop moving, and the locking portion also rotates with the driving portion to the clamping position or the releasing position to stop moving. That is, when the driving portion rotates to the fastening position where the second stop member and the first stop member are connected, the locking portion also rotates in place.

In an embodiment, the second connecting assembly includes a clamping member capable of passing through the notch and a limiting member for limiting the mounting table; one side of the clamping member is provided with a clamping portion; and there are a plurality of limiting members. The clamping member can pass through the notch, such that one side of the second connecting assembly is attached to one side of the first connecting assembly to realize combination of the placement table and the placement table positioning device. The clamping portion is configured to clamp and fasten the locking portion. When the driving portion rotates to a position where the first stop member at the second limiting end is clamped with the second stop member, the locking portion rotates synchronously, until an end of the locking portion is embedded into the clamping portion. By this time, through the first stop member and the second stop member, the locking portion is not shifted. Through engagement between the locking portion and the clamping portion, the placement table and the placement table positioning device are fixedly locked.

In an embodiment, the placement table transferring device includes a caster and a damping assembly provided on the caster; the damping assembly includes a lower supporting seat, an upper supporting seat, and a damper provided between the upper supporting seat and the lower supporting seat; the upper supporting seat is movably connected to the lower supporting seat; and the upper supporting seat is capable of rotating relative to the lower supporting seat. The placement table is provided on the placement table transferring device before positioned. The placement table transferring device moves through the caster at a bottom of the placement table transferring device, and can move the placement table to a specified position. In case of an accident, when the placement table transferring device is under a downward pressure, the damping assembly can damp an impact force of the external pressure on the support structure and the caster of the placement table transferring device, thereby protecting the placement table transferring device. The damper is accommodated and provided between the lower supporting seat and the upper supporting seat. When the placement table transferring device is under the downward external pressure, the external force is transmitted to the upper supporting seat, such that the upper supporting seat rotates, and transmits the pressure to the damper, thereby preventing damage to the caster.

In an embodiment, a positioner is provided on the placement table transferring device; and the positioner is configured to position relative positions of the placement table transferring device and the placement table positioning device. The placement table transferring device is attached and positioned with the placement table positioning device through the positioner, thereby realizing preliminary positioning on the placement table transferring device. Therefore, the first connecting assembly and the second connecting assembly can be combined more quickly in subsequent operation, which facilitates subsequent positioning of the placement table positioning device on the placement table.

In an embodiment, an opening is formed in the placement table transferring device; and the opening is configured to accommodate the clamping assembly. The first connecting assembly or the second connecting assembly on the placement table is located at the opening, such that the first connecting assembly can be aligned and matched with the second connecting assembly at the opening.

A second aspect of the present disclosure provides a placement table positioning method, including: moving the placement table transferring device to a preset position where the placement table transferring device is docked with the placement table positioning device, where the placement table is provided on the placement table transferring device, the first connecting assembly and the second connecting assembly are respectively oppositely provided on the placement table and the placement table positioning device, and by moving the placement table transferring device to the preset position, the placement table transferring device is positioned preliminarily; moving the placement table positioning device, such that the first connecting assembly and the second connecting assembly are aligned and matched, where by moving the placement table positioning device, the first connecting assembly and the second connecting assembly are attached; rotating the first connecting assembly to the clamping position, such that the first connecting assembly and the second connecting assembly are locked, where by locking the first connecting assembly and the second connecting assembly, the placement table positioning device and the placement table are fixed; and moving the placement table positioning device, and positioning a coordinate position of the placement table, where the placement table positioning device moves the placement table from an initial position to the preset coordinate position, thereby positioning the placement table.

In an embodiment, the placement table transferring device is provided with the positioner and the opening; and the moving the placement table transferring device to a preset position where the placement table transferring device is docked with the placement table positioning device includes: attaching the placement table transferring device to one side of the placement table positioning device through the positioner, and aligning and attaching the first connecting assembly and the second connecting assembly at the opening. Through the positioner, the placement table transferring device is positioned preliminarily relative to the placement table positioning device. Through the opening, the placement table positioning device moves up and down conveniently, such that the first connecting assembly can be matched with the second connecting assembly through the opening.

In an embodiment, the first connecting assembly includes the driving portion, the locking portion, and the limiting portion; the limiting portion is provided with the first stop member; the driving portion is provided with the second stop member corresponding to the first stop member; the second connecting assembly includes the clamping portion; and the rotating the first connecting assembly to the clamping position, such that the first connecting assembly and the second connecting assembly are locked includes: rotating the driving portion, until the first stop member and the second stop member are fastened, and the locking portion is rotated to the clamping portion for locking. By rotating the driving portion, the locking portion rotates with the driving portion. Through the first stop member and the second stop member, the driving portion is limited to move. After rotating in place, the driving portion and the locking portion stops rotating without shifting. This makes the locking member more stable.

A third aspect of the present disclosure provides a radiotherapy system, including a radioactive ray generation device, and the placement table positioning system.

In an embodiment, the radiotherapy system is a BNCT system, and the radioactive ray generation device is a neutron generation device.

According to the placement table positioning system provided by the first aspect of the present disclosure, through the connecting device, the placement table positioning device can be quickly locked with the placement table, which omits unnecessary positioning in treatment. Through the placement table positioning device, a coordinate position can be provided quickly and accurately to position the patient, which saves operation time before positioning, and effectively prevents the unnecessary particle irradiation dose.

According to the placement table positioning method provided by the second aspect of the present disclosure, the placement table positioning device positions the placement table through steps in the method. With the one-stop locking action between the placement table positioning device and the placement table and the simple operation, the placement table positioning method greatly saves the time for positioning the whole placement table, and makes the placement table irradiated and positioned more conveniently, quickly and accurately.

### BRIEF DESCRIPTION OF THE DRAWINGS

FIG. 1 is a schematic structural view of a BNCT system according to an embodiment;
FIG. 2 is an overall schematic structural view of a placement table positioning system according to an embodiment;
FIG. 3 is a schematic structural view of a connecting device in a placement table positioning system according to an embodiment;
FIG. 4 is a schematic structural view of a first connecting assembly in a placement table positioning system according to an embodiment;
FIG. 5 is a schematic view illustrating a state in which a connecting device in a placement table positioning system is located at a clamping position according to an embodiment;
FIG. 6 is a schematic view illustrating a state in which a connecting device in a placement table positioning system is located at a releasing position according to an embodiment;
FIG. 7 is a schematic structural view of a second connecting assembly in a placement table positioning system according to an embodiment;
FIG. 8 is a schematic view illustrating matching between a limiting member and a mounting table in a placement table positioning system according to an embodiment;
FIG. 9 is a schematic structural view of a placement table transferring device in a placement table positioning system according to an embodiment;
FIG. 10 is a schematic structural view of a damping assembly in a placement table positioning system according to an embodiment;
FIG. 11 is a schematic view illustrating a state in which a placement table transferring device and a placement table positioning device in a placement table positioning system are docked at a preset position according to an embodiment;
FIG. 12 is a partially enlarged view of FIG. 11;
FIG. 13 is a schematic view illustrating a state in which a placement table positioning device in a placement table positioning method positions a placement table according to an embodiment;
FIG. 14 is a schematic view illustrating positioning of a placement table in a BNCT system according to an embodiment;
FIG. 15 is a schematic structural view of a placement table positioning device in a BNCT system according to an embodiment;
FIG. 16 is a schematic view of FIG. 15 in another direction;
FIG. 17 is a modular schematic view of a BNCT system according to an embodiment;
FIG. 18 is a schematic view of a placement table transferring device and a transfer trolley positioning mechanism in a BNCT system according to an embodiment;
FIG. 19 is a schematic view illustrating a state in which a placement table positioning device in a BNCT system is located at different positions according to an embodiment;
FIG. 20 is a top view of FIG. 19 in a direction parallel to a ground;
FIG. 21 is a sectional view of FIG. 20 on a plane OO;
FIG. 22 is a flowchart of a placement table control method in a BNCT system according to an embodiment; and
FIG. 23 is a flowchart of a method for controlling a placement table to move away from a beam outlet in a BNCT system according to an embodiment.

In the figures:
100: BNCT system, 101: irradiation room, 102: charged particle beam generation room, 103: partition wall, 104: simulated positioning room, 1011: ceiling, 1012: floor, 10: neutron generation device, 11: accelerator, 20: beam shaping assembly (BSA), 21: reflector, 22: moderator, 23: thermal neutron absorber, 24: radiation shield, 25: beam outlet, 30: collimator, 40: placement table, 50: radiation shielding device, 200: patient, 401: placement table transferring device, 404: caster, 300: connecting device, 500: first connecting assembly, 501: mounting table, 502: locking member, 503: notch, 504: driving portion, 505: locking portion, 506: limiting portion, 507: limiting end, 508: first stop member, 509: second stop member, 510: flange connector, 700: second connecting assembly, 701: clamping member, 702: clamping portion, 703: limiting member, 800: damping assembly, 801: lower supporting seat, 802: upper supporting seat, 803: damper, 804: bump, 805: pin shaft, 806: support rod, 4011: positioner, 4012: opening, 402: transfer trolley positioning mechanism, 4021: hole, 4022: pin, 60: placement table positioning device, 61: positioning mechanism, 62: driving mechanism, 601: laser positioning device, 611: linear shaft, 612: mechanical arm, 6111: slide track, 6112: supporting seat, 70: control device, 71: user interface, 72: system control module, 73: positioning control module, 80: sensor, and 90: treatment planning device.

### DETAILED DESCRIPTION OF THE EMBODIMENTS

To make the foregoing objectives, features, and advantages of the present disclosure clearer and more comprehensible, the specific implementations of the present disclosure are described in detail below with reference to the drawings. The following describes many details in order to provide a thorough understanding of the present disclosure. However, the present disclosure can be implemented in many other ways other than those described herein, and those skilled in the art can make similar improvements without departing from the connotation of the present disclosure, and thus the present disclosure is not limited to the specific embodiments disclosed below.

In the description of the present disclosure, the terms "central", "longitudinal", "transverse", "length", "width", "thickness", "upper", "lower", "front", "back", "left", "right", "vertical", "horizontal", "top", "bottom", "inner", "outer", "clockwise", "anticlockwise", "axial", "radial" and "circumferential" etc. are used to indicate orientations shown in the accompanying drawings. It should be noted that these terms are merely intended to facilitate a simple description of the present disclosure, rather than to indicate or imply that the mentioned apparatus or elements must have the specific orientation or be constructed and operated in the specific orientation. Therefore, these terms may not be construed as a limitation to the present disclosure.

In addition, the terms "first" and "second" are merely intended for a purpose of description, and shall not be understood as an indication or implication of relative importance or implicit indication of a quantity of indicated technical features. Therefore, a feature limited by "first" or "second" may explicitly or implicitly include at least one such feature. In the descriptions about the present disclosure, "a plurality of' means at least two, for example, two or three, unless otherwise specifically limited.

In the present disclosure, unless otherwise clearly limited, the terms "installation", "interconnection", "connection" and "fixation" etc. are intended to be understood in a broad sense. For example, the "connection" may be a fixed connection, a removable connection or an integral connection; may be a mechanical connection or an electrical connection; may be a direct connection or an indirect connection using a medium; and may be a communication or an interaction between two elements, unless otherwise clearly specified and limited. Those of ordinary skill in the art may understand specific meanings of the above terms in the present disclosure based on a specific situation.

In the present disclosure, unless otherwise clearly specified and limited, when it is described that a first feature is "above" or "below" a second feature, it indicates that the first and second features are in direct contact or the first and second features are in indirect contact through a medium. In addition, when it is described that the first feature is "over", "above" and "on" the second feature, it indicates that the first feature is directly or obliquely above the second feature, or simply indicates that an altitude of the first feature is higher than that of the second feature. When it is described that the first feature is "under", "below" or "beneath" the second feature, it indicates that the first feature is directly or obliquely under the second feature or simply indicates that an altitude of the first feature is lower than that of the second feature.

It should be noted that when a component is "fixed" or "provided" on another component, the component may be fixed on the another component directly or via an intermediate component. When a component is connected to another component, the component may be connected to the another component directly or via an intermediate component. The terms "vertical", "horizontal", "upper", "lower", "left", "right" and other similar expressions used herein are for illustrative purposes only, rather than to mean the only implementation.

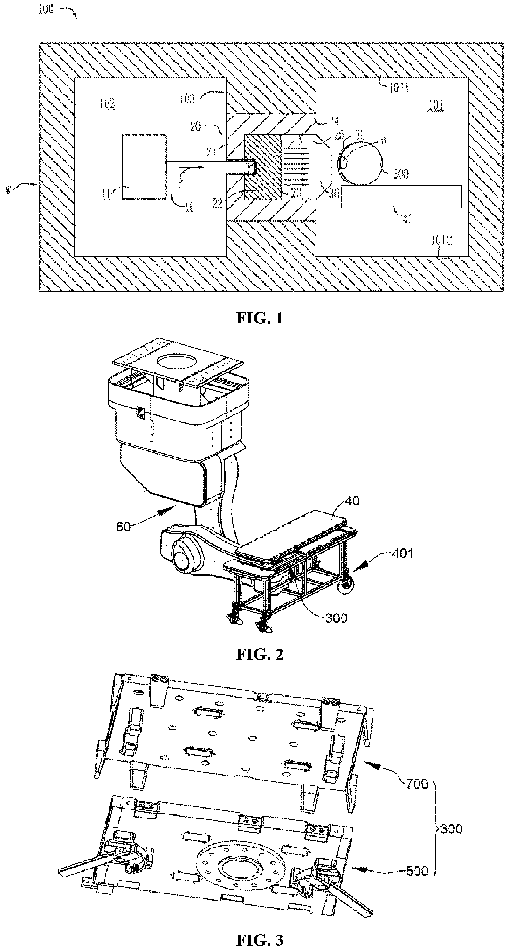

Referring to FIG. 1, FIG. 1 illustrates a radiotherapy system according to an embodiment of the present disclosure. The radiotherapy system is preferably a BNCT system 100, and includes a neutron generation device 10, a BSA 20, a collimator 30, and a placement table 40. The neutron generation device 10 includes an accelerator 11 and a target T. The accelerator 11 is configured to accelerate charged particles (such as protons and deuterons) to generate a charged particle line P such as a proton line. The charged particle line P is irradiated onto the target T and interacted with the target T to generate a neutron line (a neutron beam) N. The target T is preferably a metal target. An appropriate nuclear reaction is selected according to characteristics such as a desired neutron yield and energy, available energies of the accelerated charged particles, a current, physical and chemical properties of the metal target, or the like. Nuclear reactions commonly discussed include ⁷Li(p,n)⁷Be and ⁹Be(p,n)⁹B, both of which are endothermic reactions. Energy thresholds of the two nuclear reactions are respectively 1.881 MeV and 2.055 MeV Epithermal neutrons with an energy level of keV are considered as an ideal neutron source in the BNCT. Theoretically, if photons with energies slightly higher than the threshold are used to bombard the lithium target, neutrons with relatively low energies can be generated, and can be used clinically without excessive moderation. However, the target made of the lithium (Li) and beryllium (Be) has a small action cross section with the photons at the threshold energy. In order to generate an enough large neutron flux, photons with high energies are usually selected to trigger the nuclear reaction. The ideal target features a high neutron yield, a capability of generating neutrons with energies close to the energy range of epithermal neutrons (which will be described below in detail), no excessive long-range radiation, safety, a cheap cost, an easy operation, a high temperature resistance, etc. However, as a matter of fact, a nuclear reaction meeting all requirements cannot be found. The target made of the lithium is used in the embodiment of the present disclosure. However, as is known to those skilled in the art, the target T may also be made of a metal material other than the lithium and beryllium, such as tantalum (Ta) or tungsten (W). The target T may be a circular plate, may also be other solid shapes, and may further be a liquid (liquid metal). The accelerator 11 may be a linear accelerator, a cyclotron, a synchrotron and a synchrocyclotron. The neutron generation device 10 may be a nuclear reactor without the accelerator and the target. No matter whether a neutron source in the BNCT comes from the nuclear reactor or the nuclear reaction between the accelerated charged particles and the target, a mixed radiation field is generated as a matter of fact, that is, the generated beam includes neutrons and photons having energies from low to high. As for the BNCT on deep-seated tumors, except the epithermal neutrons, the more the remaining radioactive rays, the greater the proportion causing non-selective dose deposition in the normal tissues. Therefore, the radioactive rays causing the unnecessary dose deposition should be reduced as much as possible. In addition, for normal tissues of the irradiated body, various radioactive rays should not be excessive, so as not to cause the unnecessary dose deposition.

The neutron beam N generated by the neutron generation device 10 is irradiated onto the irradiated body 200 on the placement table 40 through the BSA20 and the collimator 30. The BSA 20 can adjust quality of the neutron beam N generated by the neutron generation device 10. The collimator 30 is configured to gather the neutron beam N, such that the neutron beam N has a high targeting ability in the treatment. The placement table 40 and the irradiated body 200 may also be adjusted, such that the beam is directed at tumor cells M in the irradiated body 200. The adjustment may be realized manually, and may also be realized automatically through a series of control mechanisms (which will be described below in detail). It may be understood that the collimator may also not be provided in the present disclosure, and the beam from the BSA 20 is directly irradiated onto the irradiated body 200 on the placement table 40.

The BSA 20 further includes a reflector 21, a moderator 22, a thermal neutron absorber 23, a radiation shield 24, and a beam outlet 25. The energy spectrum of neutrons generated by the radiation generation device 10 is very wide. Except that epithermal neutrons meet the treatment requirement, neutrons and photons of other types are to be reduced as much as possible, so as not to hurt the operator or the irradiated body. Hence, concerning neutrons from the neutron generation device 10, energies (>40 keV) of fast neutrons are adjusted by the moderator 22 to an energy range (0.5 eV to 40 keV) of the epithermal neutrons, and the thermal neutrons (<0.5 eV) are reduced as much as possible. The moderator 22 is made of a material having a large action cross section with the fast neutrons but a small action cross section with the epithermal neutrons. As a preferred embodiment, the moderator 22 is made of at least one of D₂O, AlF₃, Fluental^{™}, CaF₂, Li₂CO₃, MgF₂, and Al₂O₃. The reflector 21 surrounds the moderator 22, and reflects neutrons diffused through the moderator 22 back to the neutron beam N, thereby improving the utilization rate of the neutrons. The reflector is made of a material with a strong neutron reflectivity. As a preferred embodiment, the reflector 21 is made of at least one of Pb or Ni. The thermal neutron absorber 23 is provided behind the moderator 22, and made of a material having a large action cross section with the thermal neutrons. As a preferred embodiment, the thermal neutron absorber 23 is made of Li⁻⁶. The thermal neutron absorber 23 is configured to absorb thermal neutrons passing through the moderator 22 to reduce the thermal neutrons in the neutron beam N, thereby preventing an excessive dose to the superficial normal tissue in the treatment. It may be understood that the thermal neutron absorber may also be integrated with the moderator. The material of the moderator contains Li⁻⁶. The radiation shield 24 is configured to shield neutrons and photons leaked from a portion other than the beam outlet 25. A material of the radiation shield 24 includes at least one of a photon shielding material and a neutron shielding material. As a preferred embodiment, the material of the radiation shield 24 includes lead (Pb) as the photon shielding material and polyethylene (PE) as the neutron shielding material. The collimator 30 is provided behind the beam outlet 25. An epithermal neutron beam from the collimator 30 is irradiated onto the irradiated body 200. After passing through a superficial normal tissue of the irradiated body, the epithermal neutron beam is moderated as thermal neutrons to reach the tumor cells M. It may be understood that the BSA 20 may also be other structures, provided that the epithermal neutron beam can be obtained to meet the treatment requirement. For ease of description, when the collimator 30 is provided, an outlet of the collimator 30 may also be viewed as the beam outlet 25 hereinafter.

After the boron (B-10)-containing drug is taken by or injected into the irradiated body 200, the boron-containing drug is selectively gathered to the tumor cells M. With a large capture cross section of the boron (B-10)-containing drug for thermal neutrons, and through the ¹⁰B(n,α)⁷ Li neutron capture reaction and the nuclear fission reaction, ⁴He and ⁷Li heavy charged particles are generated. The two heavy charged particles have an average energy of about 2.33 MeV, and have characteristics of the high linear energy transfer (LET), and the short range. The alpha particle has the LET of 150 keV/µm, and the range of 8 µm. The ⁷Li heavy charged particle has the LET of 175 keV/µm, and the range of 5 µm. The total range of the two particles is approximately equivalent to a cell size, such that the radiation damage to an organism can be limited to a cell level, and a purpose of locally killing the tumor cells on premise of no serious damage to normal tissues can be achieved.

In the embodiment, a radiation shielding device 50 is further provided between the irradiated body 200 and the beam outlet 25, so as to shield radiation of the beam from the beam outlet 25 on the normal tissues of the irradiated body. It may be understood that the radiation shielding device 50 may also not be provided. The whole BNCT system 100 is accommodated in a concrete building. Specifically, the BNCT system 100 further includes an irradiation room 101 and a charged particle beam generation room 102. The irradiated body 200 on the placement table 40 is irradiated by the neutron beam N in the irradiation room 101. The accelerator 11 is accommodated in at least a portion of the charged particle beam generation room 102. At least a portion of the BSA 20 is accommodated in a partition wall 103 between the irradiation room 101 and the charged particle beam generation room 102. It may be understood that the partition wall 103 may separate the irradiation room 101 and the charged particle beam generation room 102 apart completely, and may also separate the irradiation room 101 and the charged particle beam generation room 102 apart partially, and make the irradiation room 101 and the charged particle beam generation room 102 communicate with each other. There may be one or more targets T. The charged particle line P may be interacted with one or more targets T selectively or interacted with a plurality of targets T at the same time, so as to generate one or more neutron beams N for treatment. Corresponding to a number of targets T, there may also be one or more BSAs 20, one or more collimators 30, and one or more placement tables 40. A plurality of placement tables may be provided in a same irradiation room, and may also be provided in individual irradiation rooms respectively. The irradiation room 101 and the charged particle beam generation room 102 each are a space enclosed by a concrete wall W (including the partition wall 103). The concrete structure can shield neutrons and other radioactive rays leaked in work of the BNCT system 100. The BNCT system 100 may further include a preparation room, a control room, and other spaces for auxiliary treatment (not shown in the figure). Each irradiation room may be provided with one preparation room, so as to make preparatory work before the irradiation, including injection of the boron-containing drug and simulation of a treatment plan. The control room is configured to control the accelerator, a beam transmission portion, a placement table positioning device, and the like, and control and manage the whole irradiation process. A plurality of irradiation rooms may further be monitored at the same time in the control room. The BNCT system 100 may further include a simulated positioning room 104 (which will be described below in detail) configured to perform simulated positioning on the irradiated body 200 before the irradiation. A simulated beam outlet 25' same as the beam outlet 25 is formed in the simulated positioning room 104. This omits the time for positioning the irradiated body 200 in the irradiation room 101, and improves the utilization rate of the irradiation room 101. It may be understood that the simulated positioning room may also be used as the preparation room.

The BNCT system 100 further includes a placement table positioning system. Referring to FIG. 2, FIG. 2 is an overall schematic structural view of a placement table positioning system according to an embodiment of the present disclosure. The placement table positioning system includes a placement table 40, a placement table positioning device 60, a placement table transferring device 401, and a connecting device 300. The placement table 40 is configured to bear a patient (the irradiated body). The placement table positioning device 60 is configured to move and position the placement table 40, and support the placement table 40 when moving and positioning the placement table. The placement table transferring device 401 is configured to support the placement table 40 when transferring the placement table. The connecting device 300 has a clamping position where the placement table 40 and the placement table positioning device 60 are locked and a releasing position where the placement table 40 and the placement table positioning device 60 are separated. The connecting device 300 includes a first connecting assembly 500 and a second connecting assembly 700 that cooperate with each other. The first connecting assembly 500 and the second connecting assembly 700 are respectively oppositely provided on the placement table 40 and the placement table positioning device 60.

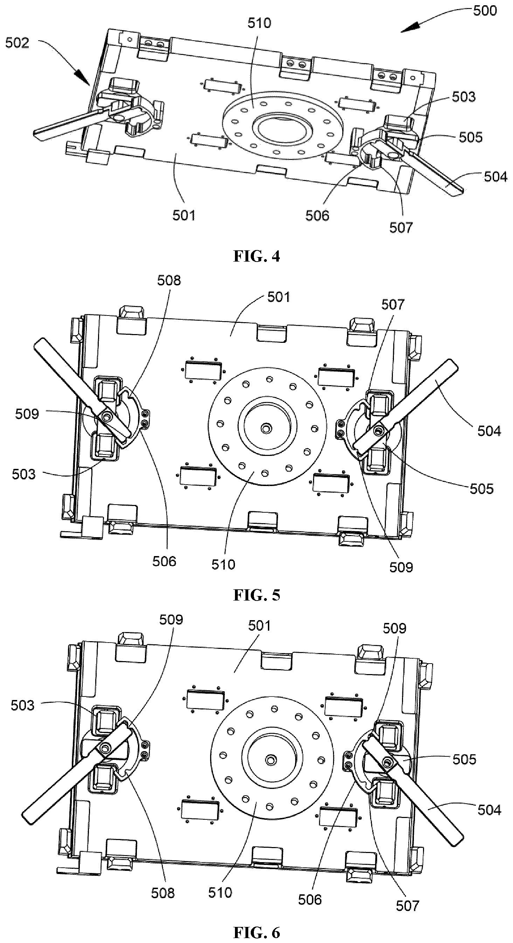

Referring to FIG. 3, FIG. 3 is a schematic structural view of a connecting device 300 in a placement table positioning system according to an embodiment of the present disclosure. The connecting device 300 is configured to lock and release the placement table 40 and the placement table positioning device 60. The first connecting assembly 500 may be provided on the placement table 40, and may also be provided on the placement table positioning device 60. Accordingly, the second connecting assembly 700 may be provided on the placement table positioning device 60, and may also be provided on the placement table 40. In response to the clamping position (referring to FIG. 5), the first connecting assembly 500 and the second connecting assembly 700 are locked, such that the placement table 40 and the placement table positioning device 60 are fixed, and the placement table 40 is positioned conveniently by the placement table positioning device 60. In response to the releasing position (referring to FIG. 6), the first connecting assembly 500 and the second connecting assembly 700 may be separated, such that the placement table 40 and the placement table positioning device 60 may also be separated from each other, and the placement table 40 moves away from the placement table positioning device 60.

Referring to FIG. 4, FIG. 4 is a schematic structural view of a first connecting assembly in a placement table positioning system according to an embodiment of the present disclosure. The first connecting assembly 500 includes a mounting table 501 and a locking member 502 provided on the mounting table 501. A notch 503 aligned and matched with the second connecting assembly 700 is formed in the mounting table 501. After rotating a preset angle, the locking member 502 can be clamped with the second connecting assembly 700 passing through the notch 503. In the embodiment, the first connecting assembly 500 is provided on the placement table positioning device 60. The mounting table 501 is configured to provide the locking member 502 and the first connecting assembly 500. A flange connector 510 is provided on a surface of one side of the mounting table 501. Through the flange connector 510, the mounting table 501 is provided on a mechanical arm 612 of the placement table positioning device 60. The mounting table 501 is shaped as a plate/block with a certain thickness. The locking member 502 is provided on the mounting table 501. The locking member 502 and the flange connector 510 are provided at a same side of the mounting table 501. A number of locking members 502 is not limited. In the embodiment, two locking members 502 are provided on the mounting table 501. Each locking member 502 is correspondingly provided with two notches 503. The notches 503 are respectively located at two sides of the locking member 502. The notch 503 can allow a portion of the second connecting assembly 700 to pass through. Rotating the locking member 502 locks the placement table positioning device 60 and the placement table 40 fixedly.

In some embodiments, the locking member 502 includes a driving portion 504 movably connected relative to the mounting table 501, a locking portion 505, and a limiting portion 506 provided on the mounting table 501. The driving portion 504 is configured to drive the locking portion 505 to rotate the second connecting assembly. The limiting portion 506 is provided with a limiting end 507 for limiting a displacement of the driving portion 504. The driving portion 504 is shaped as a handle/rod. The driving portion 504 serves as a portion for rotating the whole locking member 502. The driving portion 504 is fixedly connected to the locking portion 505 through a pin shaft. The pin shaft is rotatably connected to the mounting table 501. When the driving portion 504 rotates, the locking portion 505 rotates therewith. The locking portion 505 is shaped as a block/plate. The locking portion 505 has two working states. In one working state, the locking portion is rotated to a position away from the notch 503, namely the locking portion is perpendicular to a connecting line for two notches 503 in a same group. The locking portion 505 of the second connecting assembly is located at the releasing position, without hindering movement of the second connecting assembly 700. In the other working state, the locking portion is rotated to a position close to the notch 503, namely the locking portion is parallel to a connecting line for two notches 503 in a same group. The locking portion 505 of the second connecting assembly is located at the clamping position, and can limit the movement of the second connecting assembly 700, such that the first connecting assembly 500 and the second connecting assembly 700 are closely attached, and the placement table positioning device 60 and the placement table 40 are fixedly connected. The limiting portion 506 is arc-shaped, with an arc-shaped opening toward the driving portion 504. Two ends of the limiting portion 506 each are a limiting end 507. The limiting end 507 is configured to limit movement of the driving portion 504. Two limiting ends 507 define two working positions of the driving portion 504. At one working position, the driving portion 504 is rotated to the first limiting end 507, which corresponds to a released state of the locking portion 505, as shown in FIG. 6. At the other working position, the driving portion 504 is rotated to the second limiting end 507, which corresponding to a locked state of the locking portion 505, as shown in FIG. 5.

Referring to FIG. 5 and FIG. 6, FIG. 5 is a schematic view illustrating a state in which a connecting device in a placement table positioning system is located at a clamping position according to an embodiment, and FIG. 6 is a schematic view illustrating a state in which a connecting device in a placement table positioning system is located at a releasing position according to an embodiment. A side of the limiting end 507 close to the driving portion 504 is provided with a first stop member 508. The driving portion 504 is provided with a second stop member 509 corresponding to the first stop member 508. When the locking portion 505 is located at the clamping portion, the second stop member 509 and the first stop member 508 are connected to lock the locking portion 505 and the second connecting assembly 700. The first stop member 508 and the second stop member 509 are configured to lock the driving portion 504 at the limiting end 507. The first stop member 508 protrudes from an inner side of the limiting end 507, and may be a bump. The second stop member 509 is recessed at one end of the driving portion 504, and may be a groove. The first stop member 508 and the second stop member 509 cooperate with each other. One end of the driving portion 504 may slide freely at the inner side of the limiting portion 506. When the driving portion 504 rotates around the pin shaft until the second stop member 509 at one end of the driving portion is engaged with the first stop member 508, the driving portion 504 is limited to rotate. When the driving portion 504 is rotated until the first stop member 508 at the first limiting end is engaged with the second stop member 509, the corresponding locking portion 505 is rotated to the releasing position. When the driving portion 504 is rotated until the first stop member 508 at the second limiting end is engaged with the second stop member 509, the corresponding locking portion 505 is rotated to the clamping position.

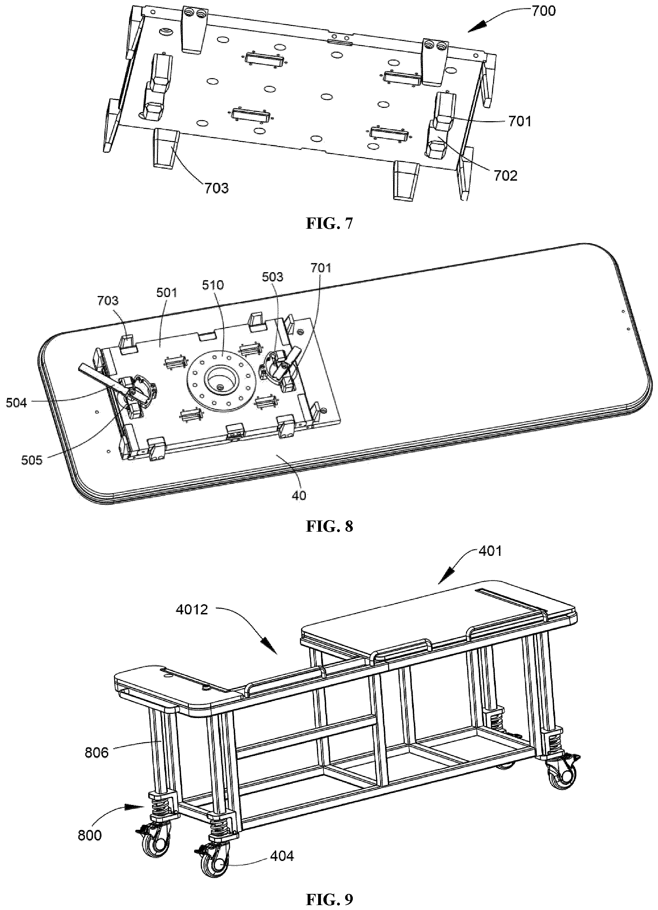

Referring to FIG. 7, FIG. 7 is a schematic structural view of a second connecting assembly in a placement table positioning system according to an embodiment of the present disclosure. The second connecting assembly 700 includes a clamping member 701 capable of passing through the notch 503 and a limiting member 703 for limiting the mounting table 501. One side of the clamping member 701 is provided with a clamping portion 702 for accommodating the locking member 502. There are a plurality of limiting members 703. In the embodiment, the second connecting assembly 700 is provided at a back of the placement table 40. There are four clamping members 701 that are respectively corresponding to four notches 503. The clamping member 701 is shaped as a strip/column having a certain height and a certain thickness. After the clamping member 701 passes through the notch 503, a portion of the clamping member 701 provided with the clamping portion 702 protrudes from the mounting table 501. The clamping portion 702 is specifically a groove. When the locking portion 505 is rotated to the clamping position, the locking portion 505 is engaged in two opposite clamping portions 702, thereby realizing locking of the locking portion 505.

Referring to FIG. 8, FIG. 8 is a schematic view illustrating matching between a limiting member 703 and a mounting table 501 in a placement table positioning system according to an embodiment. The limiting member 703 is shaped as a column. The limiting member 703 is fixed at a side edge of the second clamping assembly 700. There may be a plurality of limiting members 703. A region enclosed by the limiting members is configured to accommodate the mounting table 501. The limiting members 703 are provided corresponding to a shape of a circumference of the mounting table 501. Specifically, the mounting table 501 has a rectangular cross section. The plurality of limiting members 703 are also enclosed into a rectangle, and configured to limit the mounting table 501. When the first connecting assembly 500 is moved by the mechanical arm to a position under the second connecting assembly 700, the notch 503 in the mounting table 501 can be quickly aligned with the clamping member 701 through the limiting member 703 for insertion. A side of the limiting member 703 close to the mounting table 501 is an oblique plane. The oblique plane inclines toward a direction away from the second connecting assembly 700. In assembly of the connecting device 300, when the mounting table 501 just contacts the limiting member 703, a preset adjustment distance that is 1-5 mm preferably is formed between the mounting table 501 and the second connecting assembly 700 for the oblique plane. Consequently, the notch 503 in the mounting table 501 is aligned with the clamping member 701 more accurately, thereby facilitating quick alignment between the first connecting assembly 500 and the second connecting assembly 700.

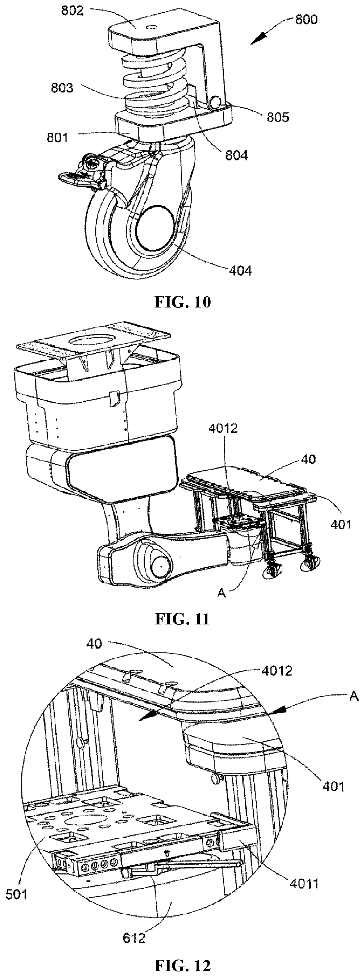

Referring to FIG. 9, FIG. 9 is a schematic structural view of a placement table transferring device in a placement table positioning system according to an embodiment of the present disclosure. The placement table transferring device 401 includes a caster 404 configured to move and a damping assembly 800 provided on the caster 404. Referring to FIG. 10, FIG. 10 is a schematic structural view of a damping assembly in a placement table positioning system according to an embodiment of the present disclosure. The damping assembly 800 includes a lower supporting seat 801, an upper supporting seat 802, and a damper 803 provided between the upper supporting seat 802 and the lower supporting seat 801. The upper supporting seat 802 is movably connected to the lower supporting seat 801. The upper supporting seat 802 is capable of rotating relative to the lower supporting seat 801. It may be understood that the upper supporting seat and the lower supporting seat may also not come into contact, but the upper supporting seat and the lower supporting seat are respectively fixed at an upper end and a lower end of the damping assembly. The placement table transferring device 401 is specifically a transfer trolley, a treatment couch trolley, etc. The placement table transferring device 401 is configured to move and transfer the placement table 40 through the caster 404. The damping assembly 800 is configured to damp a pressure on the caster 404 to protect the caster 404. The lower supporting seat 801 is shaped as a flat plate. The lower supporting seat 801 is fixed on the caster 404. A bump 804 is provided at one side of the lower supporting seat 801. The upper supporting seat 802 has an inverted L-shaped longitudinal section. The upper supporting seat 802 and the lower supporting seat 801 are combined into a C shape overall. A notch is formed in a bottom middle of the lower supporting seat 801. The notch corresponds to the bump 804. A bottom of the upper supporting seat 802 is rotatably connected to the lower supporting seat 801 through a pin shaft 805. A top of the upper supporting seat 802 is connected to a support rod 806 of the transfer trolley 401. The damper 803 takes a damping effect. The damper 803 is specifically a rectangular spring. One side of the upper supporting seat 802 and one side of the lower supporting seat 801 each are provided with a guide rod. The damper 803 surrounds the guide rod. Meanwhile, rotation of the upper supporting seat 802 toward the lower supporting seat 801 is not hindered by two guide rods. When the placement table 40 is moved by the placement table positioning device 60, in case of an operational error or other accidents, for example, the mechanical arm 612 should be raised up but is pushed down such that the transfer trolley 401 suddenly suffers a downward pressure, the pressure is transmitted to the upper supporting seat 802 through the support rod 806. The upper supporting seat 802 under the pressure rotates around the pin shaft 805, and transmits the pressure to the damper 803. The damper 803 causes compressive deformation to damp the downward pressure, thereby protecting the caster 404.

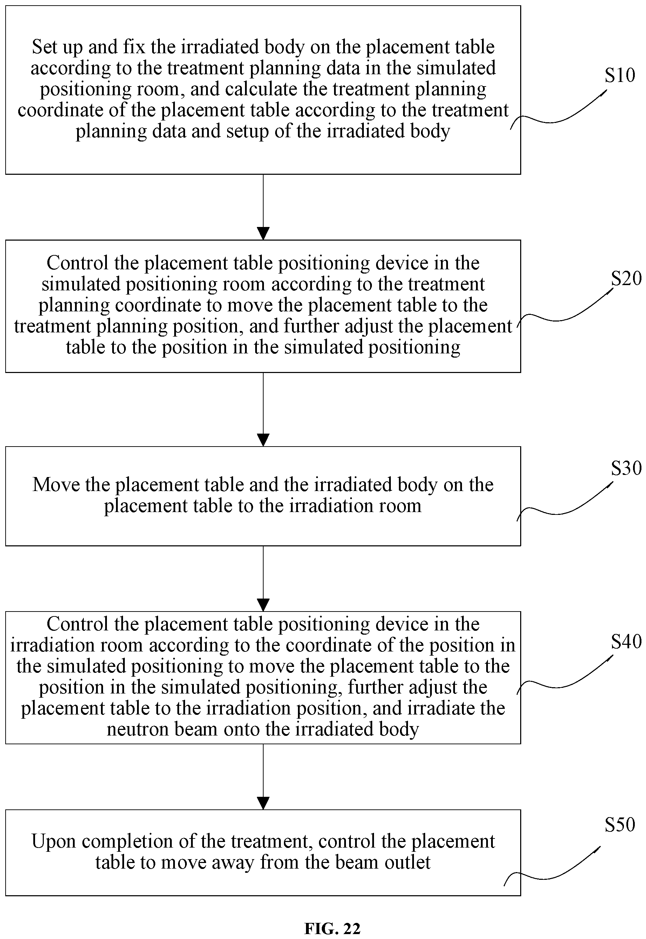

Referring to FIG. 11, FIG. 11 is a schematic view illustrating a state in which a placement table transferring device and a placement table positioning device are docked at a preset position in a placement table positioning system according to an embodiment of the present disclosure. A positioner 4011 is provided on the placement table transferring device 401. The positioner 4011 is configured to position relative positions of the placement table transferring device 401 and the placement table positioning device 60. Specifically referring to the enlarged view in FIG. 22, the positioner 4011 is L-shaped. The positioner 4011 is provided on the support rod 806 of the placement table transferring device 401. The mechanical arm 612 of the placement table positioning device 60 is connected to the mounting table 501 through the flange connector 510. By closely attaching the positioner 4011 to a side of the mounting table 501, an initial position of the placement table transferring device 401 is obtained.

In some embodiments, an opening 4012 is formed in the placement table transferring device 401. The opening 4012 is configured to accommodate the connecting device 300. When the placement table 40 is placed on the placement table transferring device 401, the second connecting assembly 700 is located at the opening 4012. The first connecting assembly 500 can pass through the opening 4012, and cooperate with the second connecting assembly 700 at the opening 4012. After the placement table transferring device 401 is positioned preliminarily through the positioner 4011, the placement table positioning device 60 and the first connecting assembly 500 move quickly to the opening 4012, and the first connecting assembly 500 and the second connecting assembly 700 are aligned and attached.

An embodiment of the present disclosure provides a placement table positioning method. As is known to those skilled in the art, the placement table positioning method is a specific method for locking the placement table positioning device and the placement table.

S11: The placement table transferring device 401 is moved to a preset position where the placement table transferring device is docked with the placement table positioning device 60. The placement table 40 is provided on the placement table transferring device 401. The first connecting assembly 500 and the second connecting assembly 700 are respectively oppositely provided on the placement table 40 and the placement table positioning device 60.

Specifically, referring to FIG. 11 and FIG. 12, the placement table transferring device 401 is provided with the positioner 4011 and the opening 4012. The positioner 4011 is L-shaped. The mechanical arm 612 is connected to the mounting table 501 through the flange connector 510. The mounting table 501 is rectangular. The L-shaped positioner 4011 is closely attached to a right-angled side edge of the mounting table 501 connected to the placement table positioning device 60. By this time, the placement table positioning device 60 and the placement table transferring device 401 are positioned preliminarily. The placement table positioning device 60 can quickly move to the opening 4012, and pass through the opening 4012, such that the first connecting assembly 500 gets close to the second connecting assembly 700. The placement table positioning device 60 drives the first connecting assembly 500 to get close to the second connecting assembly 700 quickly.

S12: The placement table positioning device 60 is moved, such that first connecting assembly 500 and the second connecting assembly 700 are aligned and attached.

Specifically, referring to FIG. 8, the first connecting assembly 500 includes the mounting table 501. The notch 503 is formed in the mounting table 501. The second connecting assembly 700 includes the clamping member 701. The placement table transferring device 401 keeps static after positioned initially. The placement table positioning device 60 is moved to the opening 4012. A plurality of limiting members 703 are arranged at the side edge of the second connecting assembly 700. By raising up the mechanical arm 612, the mounting table 501 is moved to a region enclosed by the limiting members 703. The clamping member 701 passes through the notch 503. By raising up the mechanical arm 612 continuously, the first connecting assembly 500 gets close to the second connecting assembly 700 gradually, until being attached to the second connecting assembly.

S13: The first connecting assembly 500 is rotated to the clamping position, such that the first connecting assembly 500 and the second connecting assembly 70 are locked.

Specifically, referring to FIG. 8, the first connecting assembly 500 includes the driving portion 504, the locking portion 505, and the limiting portion 506. The limiting portion 506 is provided with the first stop member 508. The driving portion 504 is provided with the second stop member 509 corresponding to the first stop member 508. The second connecting assembly 700 includes the clamping member 701. The clamping portion 702 is provided on the clamping member 701. The driving portion 504 is rotated, and the locking portion 505 is also rotated therewith. The driving portion 504 is rotated, until the first stop member 508 and the second stop member 509 are fastened, and the locking portion 505 is rotated between the clamping portions 702 for locking.

S14: The placement table positioning device 60 is moved, and a coordinate position of the placement table 40 is positioned.

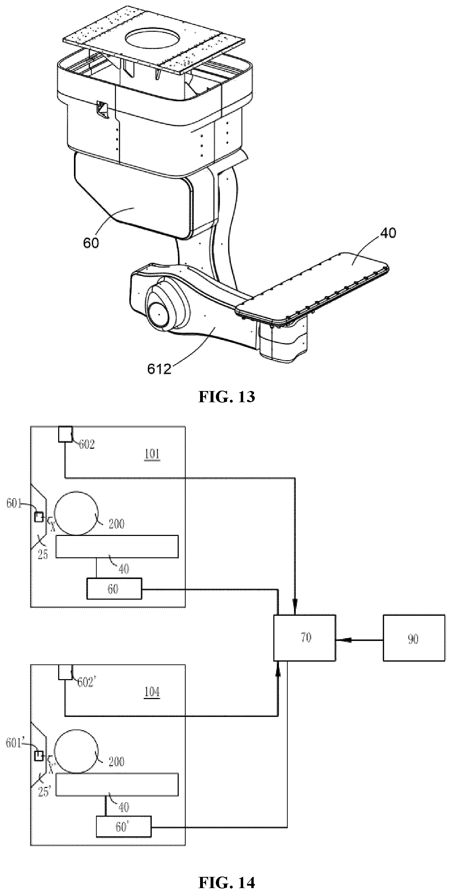

Specifically, referring to FIG. 13, in the irradiation room, a control device 70 controls the mechanical arm 612 according to a coordinate position determined in simulated positioning to move the placement table 40 to the position determined in simulated positioning. An irradiation position is determined to irradiate a patient 200.

S15: The first connecting assembly 500 is rotated to the releasing position, such that the first connecting assembly 500 and the second connecting assembly 700 are separated.

Specifically, referring to FIG. 6, upon completion of irradiation, the control device 70 controls the placement table positioning device 60 to move the placement table 40 to the placement table transferring device 401. The driving portion 504 is rotated reversely, and the locking portion 505 is also rotated therewith. The driving portion 504 is rotated, until the first stop member 508 at the other end of the limiting portion 506 and the second stop member 509 on the driving portion 504 are fastened, and the locking portion 505 is rotated to a position away from the clamping portion 702.

Referring to FIG. 14, the BNCT system 100 further includes the control device 70. The placement table 40 and the irradiated body 200 on the placement table 40 are supported by the placement table positioning device 60. The control device 70 is connected to the placement table positioning device 60 and can be configured to control the placement table positioning device 60. The control device 70 can further be connected to the neutron generation device 10 and can be configured to control the neutron generation device 10 to irradiate the neutron beam N onto the irradiated body 200 on the placement table 40. In the embodiment, the irradiation room 101 and the simulated positioning room 104 are respectively provided with the placement table positioning device 60 and the placement table positioning device 60' that are the same, and respectively have a same positional relationship with the beam outlet 25 and the simulated beam outlet 25'. That is, a coordinate system XYZ for operating the placement table 40 and the placement table positioning device 60 in the irradiation room 101 and a coordinate system XYZ for operating the placement table and the placement table positioning device 60' in the simulated positioning room 104 are the same, and a reference point at a certain distance away from a center of each of the beam outlet 25 and the simulated beam outlet 25' along the neutron beam N serves as an origin. Through the placement table positioning device 60 and the placement table positioning device 60', the placement table 40 and the irradiated body 200 on the placement table 40 are subjected to simulated positioning and irradiation positioning. With the same placement table positioning devices, the irradiation positioning is more convenient, quicker and more accurate. For ease of description, only the structure of the placement table positioning device 60 in the irradiation room 101 is described below in detail.

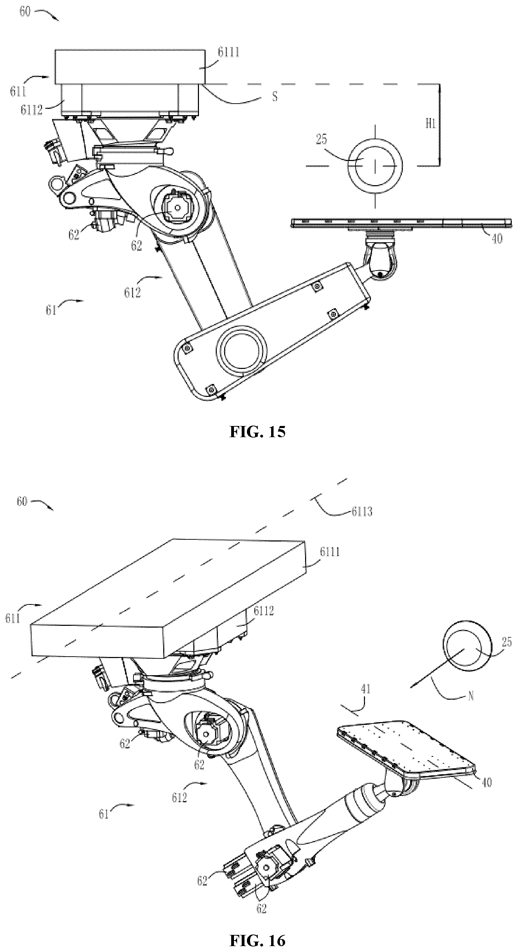

As shown in FIG. 15 to FIG. 17, in an embodiment, the placement table positioning device 60 includes a positioning mechanism 61. The positioning mechanism 61 includes a linear shaft 611 and the mechanical arm 612. The mechanical arm 612 is provided between the linear shaft 611 and the placement table 40. The placement table 40 is connected to the linear shaft 611 through the mechanical arm 612. The placement table 40 and the mechanical arm 612 can be translated together along the linear shaft 611. In the embodiment, the linear shaft 611 is provided on a ceiling 1011 of the irradiation room 101. The whole mechanical arm 612 extends toward a floor 1012 of the irradiation room 101. It may be understood that the linear shaft 611 may also be provided on other surfaces, such as the wall or the floor. The linear shaft 611 includes a slide track 6111 fixed on the ceiling 1011 and a supporting seat 6112 connected to the mechanical arm 612. The supporting seat 6112 slides along the slide track 6111. It may be understood that the linear shaft may also be other structures. The linear shaft is directly fixed on the ceiling 1011, and an additional fixing mechanical for the linear shaft, such as a steel portal frame, turns out to be unnecessary. This reduces the usage of steel in the irradiation room, and prevents the fixing mechanism from being activated by neutrons to cause secondary radiation. The mechanical arm 612 is a multiaxial mechanical arm connected to the supporting seat 6112 and the placement table 40. The placement table positioning device 60 further includes a driving mechanism 62 to drive the linear shaft 611 and the mechanical arm 612 to move. The driving mechanism 62 is controlled by the control device 70. An extension direction 6113 of the linear shaft 611 is parallel to the neutron beam N emitted from the beam outlet 25 and irradiated onto the irradiated body on the placement table 40. In the positioning process of the placement table, the whole mechanical arm 612 is translated along a direction parallel to the neutron beam N. The mechanical arm is largely located in a space between the slide track and the neutron beam outlet. This prevents each component of the mechanical arm from being activated by the neutrons to cause radiation and reduce the service life. A distance H1 from a sliding surface S between the slide track 6111 and the supporting seat 6112 to the center of the beam outlet 25 in a direction perpendicular to the sliding surface S is less than 2 m. This provides an enough operating space for the placement table positioning device 60, thereby positioning the placement table 40 at a desired position relative to the beam outlet 40. In the embodiment, the sliding surface S is parallel to a plane of the ceiling. It may be understood that the placement table positioning device 60 may further have other structures. For example, the linear shaft 611 is not provided, but the placement table 40 is connected to the mechanical arm 612 and supported by the mechanical arm 612. Alternatively, the mechanical arm 612 includes more or less arms.

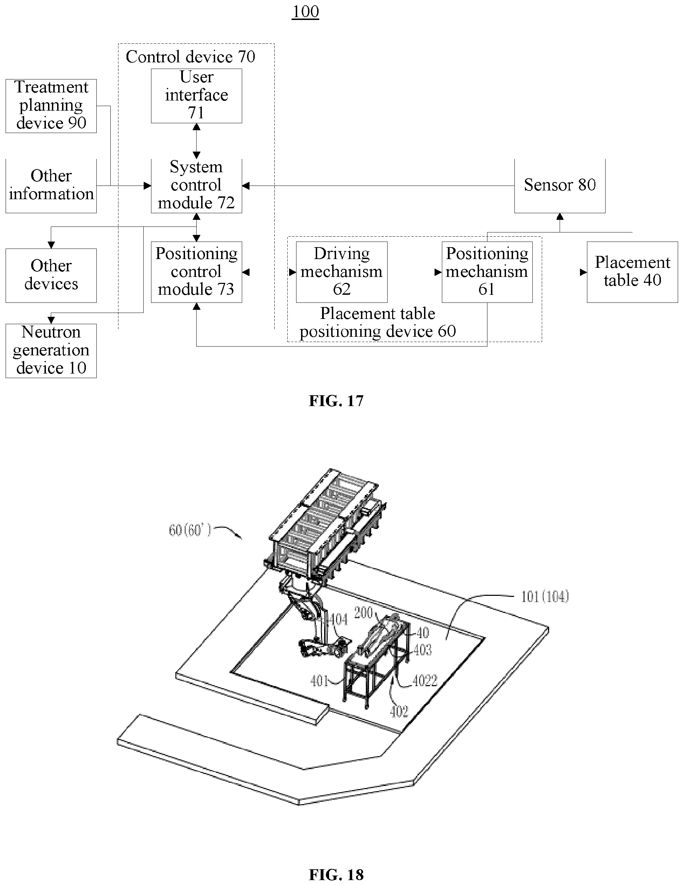

A sensor 80 may be provided on the placement table 40 or the placement table positioning device 60. As shown in FIG. 17, the sensor 80 is provided on the positioning mechanism 61 and the placement table 40. In an embodiment, the sensor 80 is an anti-collision sensor provided on the placement table 40 and the mechanical arm 612. When the placement table or an edge of the mechanical arm contacts other objects or other objects fall within a preset range of the sensor, the sensor is triggered to send a signal to the control device 70. The control device 70 controls the driving mechanism 62 to stop driving the positioning mechanism 61 to move, namely the control device controls the placement table 40 to stop moving. The anti-collision sensor may be a mechanical sensor, a photoelectric sensor, a radar sensor, an ultrasonic sensor, a laser rangefinder, etc. It may be understood that the anti-collision sensor may also send a body sensing signal, such that the driving mechanism can be manually controlled according to a sensed signal. The anti-collision sensor may also not control the placement table to stop moving, but executes other safety operations, such as an inverse movement before collision.

The control device 70 includes at least a user interface 71, so as to allow the operator to interactively control the placement table positioning device 60. The control device 70 further includes a system control module 72 and a positioning control module 73. The user interface 71 is connected to the system control module 72. The system control module 72 is connected to the positioning control module 73. The positioning control module 73 is connected to the driving mechanism 62 and configured to control the driving mechanism 62. After the system control module 72 receives an instruction from the user interface 71, the system control module 72 transmits the instruction to the positioning control module 73. The positioning control module 73 automatically controls movement of the positioning mechanism 61. Positional information of the positioning mechanism 61 may be fed back to the system control module 72 through the positioning control module 73 and transmitted to the user interface 71 for state indication. An operating state or operating data of the driving mechanism 62 is also fed back to the system control module 72 through the positioning control module 73. The system control module 72 or the positioning control module 73 controls the driving mechanism 62 according to these information. The system control module 72 may also transmit these information to the user interface 71 for state indication. The sensor 80 is also connected to the system control module 72. After receiving a signal from the senor 80, the system control module 72 transmits an instruction to the positioning control module 73 to control the movement of the placement table positioning device 60, and transmits the signal from the sensor 80 to the user interface 71 for state indication. It may be understood that the system control module 72 and the positioning control module 73 may be integrated together, and may also be provided with other hardware devices.

The BNCT system 100 further includes a treatment planning device 90. The treatment planning device 90 is configured to perform dose simulation and dose calculation according to a parameter of the neutron beam N generated by the neutron generation device 10 and medical image data of an irradiated site, and generate a treatment plan (through a Monte Carlo simulation program). According to the treatment plan, a position of the irradiated site relative to the neutron generation device 10 in irradiation and corresponding irradiation time can be determined. The control device 70 (system control module 71) is connected to the treatment planning device 90 and configured to receive treatment planning data, thereby controlling movement of the placement table positioning device 60 and the placement table positioning device 60' and the neutron beam N of the neutron generation device 10 according to the treatment planning data.

Before the irradiation, simulated positioning is performed on the irradiated body 200 according to a preformulated treatment plan of the treatment planning device 90 in the simulated positioning room 104. First of all, the placement table positioning device 60' is connected to the placement table 40, as shown in FIG. 18. In the embodiment, the placement table 40 is provided on the placement table transferring device 401. The transfer trolley 401 is positioned by a transfer trolley positioning mechanism 402 in the simulated positioning room 104 (irradiation room 101). At least two holes 4021 (not shown in the figure) are formed in a ground of the simulated positioning room 104 (irradiation room 101). At least two pins 4022 are provided correspondingly on the transfer trolley 401. The pins 4022 are respectively inserted into the holes 4021 for positioning. It may be understood that the transfer trolley 401 may also be positioned in other manners. A relative position of the placement table 40 on the transfer trolley 401 is also determined. For example, the position of the placement table 40 is limited through a limiting mechanism 403 (such as a bump on the transfer trolley). Hence, when the placement table 40 is placed on the well-positioned transfer trolley 401, the position of the placement table relative to the simulated positioning room 104 (irradiation room 101) is determined. By this time, the placement table 40 is located at an initial position A (with a same positional relationship as the irradiation room 101) in the simulated positioning room 104. The control device 70 controls the placement table positioning device 60' in the simulated positioning room 104 to move to a position where the placement table positioning device can be connected to the placement table 40, and allows the connecting device 300 to lock the placement table positioning device 60' and the placement table 40, specifically as shown in FIG. 11 and FIG. 12. Through the positioner 4011 on the support rod 806, positioning between the placement table positioning device 60' and the placement table 40 is realized. The placement table positioning device 60' is moved to a side of the mounting table 501 and closely attached to the positioner 4011. The placement table positioning device 60' can be quickly connected to the placement table.

Then, the irradiated body 200 is placed on the placement table 40. At the position of the irradiated site determined according to the preformulated treatment plan and relative to the neutron generation device 10 in the irradiation, the irradiated body 200 is set up and fixed. According to the setup at this time, the treatment planning device 90 or the control device 70 calculates the coordinate position of the placement table 40 determined in the treatment plan. By scanning the irradiated body and the placement table through CT or optical scanning, relative positions of the irradiated body and the placement table can be determined. According to the position of the irradiated site determined according to the preformulated treatment plan and relative to the neutron generation device 10 in the irradiation, a coordinate of the placement table determined in the treatment plan is calculated. It may be understood that the coordinate position of the placement table 40 may also be calculated in other manners.

According to the calculated coordinate, the control device 70 automatically controls the placement table positioning device 60' to move the placement table 40 from the initial position A to the coordinate position (treatment planning position B). After the placement table 40 moves to the coordinate position (treatment planning position B), further adjustment may be made through the user interface 71 as required to determine a position C in the simulated positioning. In case of an error in movement of the placement table 40 and the placement table positioning device 60', a movement path of the placement table positioning device 60' is recalculated or a treatment plan is regenerated. By automatically calculating the coordinate of the treatment planning position of the placement table, and automatically controlling the placement table through the placement table positioning device to move to the treatment planning position, the positioning is highly accurate and rapid.

Next, an instruction on completion of the simulated positioning is sent through the user interface 71. The control device 70 records the coordinate position (position C in the simulated positioning), and controls the placement table positioning device 60 to move the placement table 40 back to the initial position A (the placement table 40 is fittingly placed on the positioned transfer trolley 401). The control device 70 controls the connecting device 300 to unlock and release the placement table 40, and controls the placement table positioning device 60 to move to a position where the placement table positioning device is separated from the placement table 40. The transfer trolley positioning mechanism 402 is released. The transfer trolley 401 is used to transport the placement table 40 and the irradiated body 200 to the irradiation room 101.

Irradiation positioning is performed in the irradiation room 101. The transfer trolley 401 is provided with the same transfer trolley positioning mechanism 402 in the irradiation room 101 as the simulated positioning room 104. That is, the transfer trolley 401 in the irradiation room can be positioned by the transfer trolley positioning mechanism 402 according to a same fixing position in the simulated positioning room 104. The placement table positioning device 60 in the irradiation room 101 is controlled to move to a position (initial position A) where the placement table positioning device is connected to the placement table 40. The connecting device 300 is controlled to lock the placement table positioning device 60 and the placement table 40. According to the coordinate position determined in the simulated positioning, the control device 70 controls the placement table 40 move to the position C in the simulated positioning. Further adjustment may be made through the user interface 71 as required, thereby determining an irradiation position D. The operator leaves away from the irradiation room 101. The transfer trolley positioning mechanism 402 is released to move out the transfer trolley 401. With the simulated positioning in the simulated positioning room 104, the working time for positioning the irradiated body 200 before the irradiation in the irradiation room 101 is omitted. While the simulate positioning is performed, the irradiation may be performed on another irradiated body to improve the utilization rate of the device. In an embodiment, after each of the placement table positioning device 60 and the placement table positioning device 60' is locked with the placement table 40, when the placement table 40 moves from the initial position A, each of the placement table positioning device 60 and the placement table positioning device 60' may be controlled to raise up the placement table 40. After the transfer trolley positioning mechanism 402 is released to move out the transfer trolley 401, the placement table positioning device 60 and the placement table positioning device 60' each are controlled to make a further movement. This prevents interference between each of the placement table positioning device 60 and the placement table positioning device 60' and the transfer trolley 401.

In some embodiments, a laser positioning device 601 and a laser positioning device 601' that are the same and have a same positional relationship are respectively provided in the irradiation room 101 and the simulated positioning room 104. According to a position of a laser beam from the laser positioning device on the irradiated body 200, a marker is labeled on the irradiated body 200. According to the marker, the position of the irradiated body 200 in each of the simulated positioning room 104 and the irradiation room 101 is adjusted or verified, so as to ensure that the irradiated body 200 has a same position in the simulated positioning room 104 and the irradiation room 101. With the laser positioning device, the positioning is more convenient and quicker.

According to the laser beam from the laser positioning device 601 and the laser beam from the laser positioning device 601', same positions toward a central axis X of the beam outlet 25 and a central axis X' of the beam outlet 25' may further be determined respectively. As shown in FIG. 14, the position of the laser beam from the laser positioning device 60 on the irradiated body 200 and the position of the laser beam from the laser positioning device 60' on the irradiated body respectively represents a position where the central axis of the beam from the beam outlet 25 enters the irradiated body 200 and a position where the central axis of the beam from the beam outlet 25' enters the irradiated body. According to a point of incidence of the central axis of the simulated beam in the treatment plan on the voxel prosthesis tissue model, a marker is labeled on the irradiated body 200, such that the incident position of the beam determined in the simulated position and the irradiation is more accurate.

An optical verification device 602 and an optical verification device 602' that are the same and have a same positional relationship may further be respectively provided in the irradiation room 101 and the simulated positioning room 104, so as to acquire the position of the placement table 40 and an image of the irradiated body 200. Data is transmitted to the system control module 72, and compared with the treatment plan and other information. According to a compared result, adjustment is made or other treatment control operations are performed. The system control module 72 may further receive other data and information, such as data of the neutron generation device and information of the irradiated body, and controls the neutron generation device and other devices.

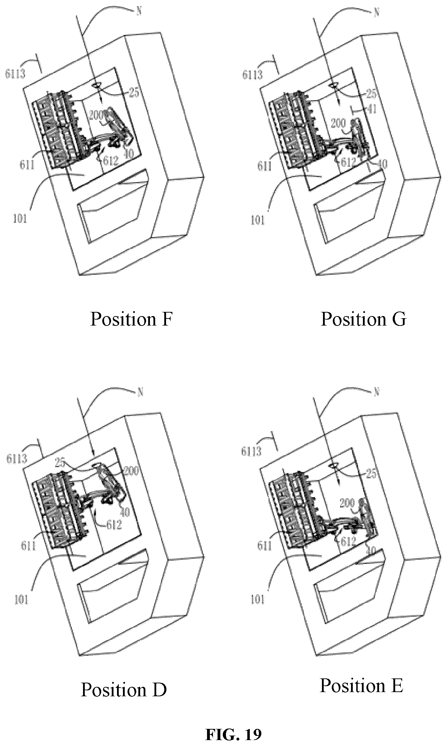

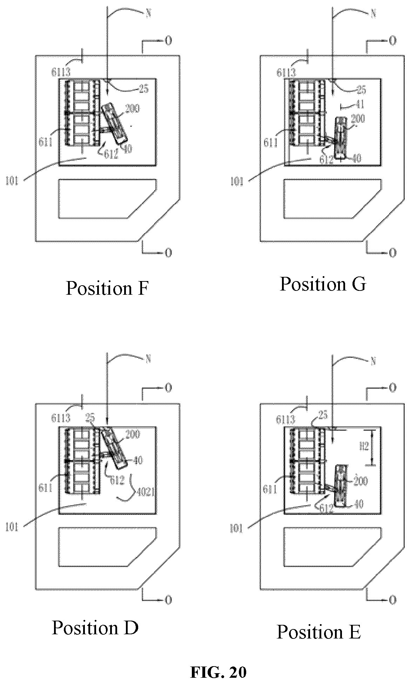

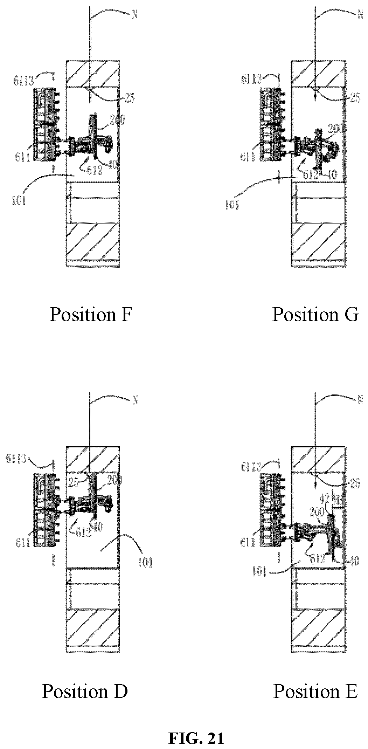

After the placement table 40 and the irradiated body 200 are adjusted well, the placement table 40 is located at the irradiation position D. An irradiation starting instruction is sent by the operator through the user interface 71. After determining that an irradiation starting condition is met, the system control module 72 controls the neutron generation device 10 to start generating the neutron beam N to irradiate the irradiated body 200 on the placement table 40. After predetermined irradiation time (such as irradiation time determined by the treatment planning data), the system control module 72 controls the neutron generation device 10 to stop irradiating the neutron beam N onto the irradiated body 200 on the placement table 40, and transmits information to the user interface 71 for state indication on completion of the treatment. Upon the completion of the treatment, the system control module 72 sends an instruction to the positioning control module 73, thereby controlling the placement table positioning device 60 to move the placement table 40 from the irradiation position D to an ending position E. The placement table 40 is away from the beam outlet 25. After the irradiation of the neutron beam N is stopped, there are still many residual radioactive rays at the beam outlet 25. By moving the placement table 40 to the position away from the beam outlet 25, the irradiated body 200 cannot be irradiated by the residual radioactive rays continuously upon the completion of the treatment, thereby reducing an unnecessary radiation dose. FIG. 19 to FIG. 21 each are a schematic view illustrating a state in which a placement table 40 and a placement table positioning device 60 are located at different positions upon completion of the treatment. First of all, upon the completion of the treatment, the linear shaft 611 is controlled, such that the placement table 40 moves away from the beam outlet 25 from the irradiation position D to a first middle position F along a direction parallel to the extension direction 6113 of the linear shaft 611. By quickly moving the placement table 40 away from the beam outlet 25, continuous irradiation of the residual radioactive rays on the irradiated body 200 upon the completion of the treatment is prevented to the greatest extent. Then, the mechanical arm 612 is controlled, such that the placement table 40 moves to a second middle position G where an extension direction 41 of the placement table 40 is basically parallel to the extension direction 6113 of the linear shaft 611. This prevents the placement table 40 from interfering the transfer trolley or obstructing an exit of a shielding door of the irradiation room 101 at the ending position E, and provides convenience for the irradiated body 200 to move away from the irradiation room 101. At last, the mechanical arm 612 is controlled, such that the placement table 40 moves close to the ground from the second middle position G to the ending position E. This is convenient for the irradiated body 200 to move away from the irradiation room 101. After the irradiated body 200 moves away, the connecting device 300 may further be unlocked, and the placement table 40 is sent back to the simulated positioning room 104. Alternatively, the connecting device 300 is unlocked, and the irradiated body 200 and the placement table 40 are sent out of the irradiation room 101 together. After the irradiated body 200 moves away from the placement table 40, the placement table 40 is sent back to the simulated positioning room 104, for example, through the transfer trolley 401. By this time, the ending position E may be the same as the initial position A. The transfer trolley 401 is also positioned through the transfer trolley positioning mechanism 402 in the irradiation room 101. The mechanical arm 612 moves the placement table 40, until the placement table 40 is fittingly located at the initial position A (ending position E) on the transfer trolley 401. At the ending position E, a minimum distance H2 from the placement table 40 to a plane of the center of the beam outlet 25 perpendicular to the neutron beam N is not less than 2,500 mm, so as to prevent high-dose irradiation of the residual radioactive rays. At the ending position E, the bearing surface 42 of the placement table 40 has a height H3 of not greater than 600 mm above the ground, so as to transfer the irradiated body 200 or the placement table 40.

Upon the completion of the treatment, the system control module 72 may automatically control the placement table 40 to move away from the beam outlet 25 according to a signal of reaching the irradiation time or a signal of stopping irradiating the neutron beam N. Alternatively, an instruction that the placement table moves away from the beam outlet may be input into the user interface 71 according to the state indication on the completion of the treatment on the user interface 71, for example, through a corresponding button on a man-machine interaction control interface 713. Then, the system control module 72 controls the placement table 40 according to the instruction to move away from the beam outlet 25.

As shown in FIG. 22, in brief, the placement table control method in the embodiment includes the following steps:
S10: The placement table positioning device 60' in the simulated positioning room 104 is connected to the placement table 40 and locked with the placement table 40 in the simulated positioning room 104, the irradiated body 200 is set up and fixed on the placement table 40 according to the treatment planning data, and the treatment planning coordinate of the placement table 40, namely a coordinate of the treatment planning position B, is calculated according to the treatment planning data and setup of the irradiated body 200.
S20: The placement table positioning device 60' is controlled according to the treatment planning coordinate to move the placement table 40 to the treatment planning position B, the placement table is further adjusted to the position C in the simulated positioning as required, and a coordinate of the position C in the simulated positioning is recorded. It may be understood that the position C in the simulated positioning may also be the treatment planning position B.
S30: The placement table positioning device 60' and the placement table 40 are unlocked, the placement table 40 and the irradiated body 200 on the placement table 40 are moved to the irradiation room 101, and the placement positioning device 60 in the irradiation room 101 is connected to the placement table 40 and locked with the placement table 40.
S40: The placement table positioning device 60 is controlled according to the coordinate of the position C in the simulated positioning to move the placement table 40 to the position C in the simulated positioning, the placement table is further adjusted to the irradiation position D as required, and the neutron beam N is irradiated onto the irradiated body 200. It may be understood that the irradiation position D may also be the position C in the simulated positioning.
S50: Upon completion of the treatment, namely after the irradiation of the neutron beam N onto the irradiated body 200 is stopped, the placement table positioning device 60 is controlled to move the placement table 40 to the ending position E, namely the placement table 40 is controlled to move away from the beam outlet 25.

It may be understood that the step S20 may further include: In case of an error when the placement table 40 moves to the treatment planning position B, a movement path of the placement table positioning device 60' is recalculated or a treatment plan is regenerated.

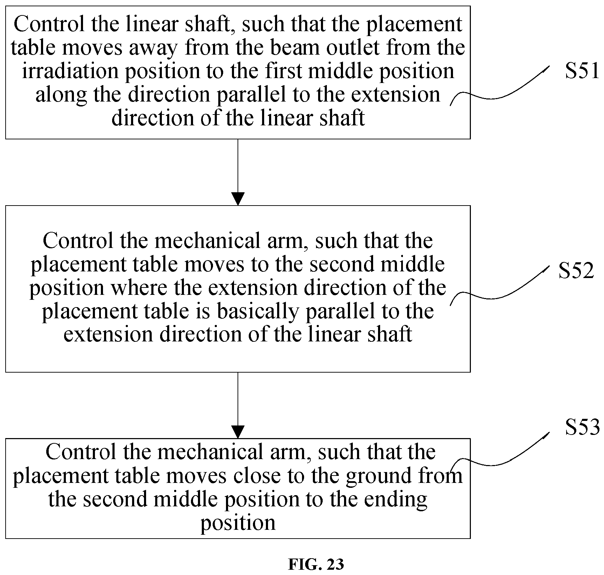

As shown in FIG. 23, specifically, the method that the placement table 40 is controlled to move away from the beam outlet 25 in the step S50 further includes:
S51: The linear shaft 611 is controlled, such that the placement table 40 moves away from the beam outlet 25 from the irradiation position D to the first middle position F along the direction parallel to the extension direction 6113 of the linear shaft 611.
S52: The mechanical arm 612 is controlled, such that the placement table 40 moves to the second middle position G where the extension direction 41 of the placement table 40 is basically parallel to the extension direction 6113 of the linear shaft 611.
S53: The mechanical arm 612 is controlled, such that the placement table 40 moves close to the ground from the second middle position G to the ending position E.

The instruction input on the user interface 71 to move the placement table away from the beam outlet may be realized by a button. The steps S51-S53 are executed automatically and continuously or the steps S51-S53 are executed one by one through three buttons respectively corresponding to the steps S51-S53. Other manners may also be available herein. It may be understood that in the step S51 and the step S53, the mechanical arm 612 may also be controlled first such that the placement table 40 moves close to the ground, and then the linear shaft 611 is controlled such that the placement table 40 moves away from the beam outlet 25 along the direction parallel to the extension direction 6113 of the linear shaft 611. The step S52 may also be after the step S53. Alternatively, the linear shaft 611 and the mechanical arm 612 are controlled at the same time such that the placement table 40 moves to the ending position E. It may be understood that according to a specific demand, the height of the placement table at the ending position E relative to the ground may also change compared with the height of the placement table at the irradiation position D relative to the ground.

The positions A-G are on the basis of a preset reference point on the placement table 40. It may be understood that the transfer trolley may also not be provided, the simulated positioning room and the irradiation room are provided with the same placement table, the irradiated body is set up in a same manner (such as through the positioning mechanism on the placement table) in the simulated positioning room and the irradiation room, and the same initial position is determined (such as through the laser positioning device).

In the embodiment, the concrete wall is a boron-containing barite concrete wall with a thickness of 1 m or more and a density of 3 g/c.c. The boron-containing concrete has better neutron absorption performance, can enhance the radiation shielding effect of the concrete, and can further reduce the neutron exposure on the metal material in the concrete. It may be understood that the concrete wall may also have other thicknesses or densities or be replaced by other materials, and different portions of the concrete wall may also correspond to different thicknesses, densities or materials. It may be understood that the present disclosure may also be applied to neutron irradiation systems of other types, and may further be applied to other radioactive ray irradiation systems such as a proton therapy system and a heavy ion therapy system. The neutron generation device may be replaced by other radioactive ray generation devices. The concrete material may be replaced as required. The placement table may also be other placement tables for the irradiated body.

The technical characteristics of the above embodiments can be employed in arbitrary combinations. To provide a concise description of these embodiments, all possible combinations of all the technical characteristics of the above embodiments may not be described; however, these combinations of the technical characteristics should be construed as falling within the scope defined by the specification as long as no contradiction occurs.

The above described are merely several embodiments of the present invention. Although these embodiments are described specifically and in detail, they should not be construed as a limitation to the patent scope of the present disclosure. It should be noted that those of ordinary skill in the art can further make several variations and improvements without departing from the concept of the present disclosure, and all of these fall within the protection scope of the present disclosure. Therefore, the protection scope of the present disclosure should be subject to the appended claims.

## Claims

1. A placement table positioning system, comprising:
a placement table configured to bear a patient;
a placement table positioning device configured to move and position the placement table;
a placement table transferring device configured to support and move the placement table; and
a connecting device having a clamping position where the placement table and the placement table positioning device are locked and a releasing position where the placement table and the placement table positioning device are separated, wherein the connecting device comprises a first connecting assembly and a second connecting assembly that cooperate with each other; and the first connecting assembly and the second connecting assembly are respectively oppositely provided on the placement table and the placement table positioning device.

2. The placement table positioning system according to claim 1, wherein the first connecting assembly comprises a mounting table and a locking member provided on the mounting table; and the locking member is configured to lock the second connecting assembly and the mounting table.

3. The placement table positioning system according to claim 2, wherein the locking member comprises a driving portion movably connected relative to the mounting table, a locking portion, and a limiting portion provided on the mounting table; the driving portion is configured to drive the locking portion to rotate; and the limiting portion is provided with a limiting end for limiting the driving portion.

4. The placement table positioning system according to claim 3, wherein a side of the limiting end close to the driving portion is provided with a first stop member; the driving portion is provided with a second stop member corresponding to the first stop member; and when the locking portion is located at the clamping position, the second stop member and the first stop member are connected.

5. The placement table positioning system according to claim 2, wherein a notch aligned and matched with the second connecting assembly is formed in the mounting table.

6. The placement table positioning system according to claim 5, wherein the second connecting assembly comprises a clamping member capable of passing through the notch and a limiting member for limiting the mounting table; one side of the clamping member is provided with a clamping portion; and there are a plurality of limiting members.

7. The placement table positioning system according to claim 6, wherein a side of the limiting member close to the mounting table is an oblique plane.

8. The placement table positioning system according to claim 1, wherein the placement table transferring device comprises a caster and a damping assembly provided on the caster; the damping assembly comprises a lower supporting seat, an upper supporting seat, and a damper provided between the upper supporting seat and the lower supporting seat; the upper supporting seat is movably connected to the lower supporting seat; and the upper supporting seat is capable of rotating relative to the lower supporting seat.

9. The placement table positioning system according to claim 1, wherein a positioner is provided on the placement table transferring device; and the positioner is configured to position relative positions of the placement table transferring device and the placement table positioning device.

10. The placement table positioning system according to claim 9, wherein the positioner is L-shaped.

11. The placement table positioning system according to claim 1, wherein an opening is formed in the placement table transferring device; and the opening is configured to accommodate the connecting device.

12. A placement table positioning method using the placement table positioning system according to any one of claims 1 to 11, comprising:
moving the placement table transferring device to a preset position where the placement table transferring device is docked with the placement table positioning device, wherein the placement table is provided on the placement table transferring device, and the first connecting assembly and the second connecting assembly are respectively oppositely provided on the placement table and the placement table positioning device;
moving the placement table positioning device, such that the first connecting assembly and the second connecting assembly are aligned and attached;
rotating the first connecting assembly to the clamping position, such that the first connecting assembly and the second connecting assembly are locked; and
moving the placement table positioning device, and positioning a coordinate position of the placement table.

13. The placement table positioning method according to claim 12, wherein the placement table transferring device is provided with the positioner and the opening; and the moving the placement table transferring device to a preset position where the placement table transferring device is docked with the placement table positioning device comprises: attaching the placement table transferring device to one side of the placement table positioning device through the positioner, and aligning and attaching the first connecting assembly and the second connecting assembly at the opening.

14. The placement table positioning method according to claim 12, wherein the first connecting assembly comprises the driving portion, the locking portion, and the limiting portion; the limiting portion is provided with the first stop member; the driving portion is provided with the second stop member corresponding to the first stop member; the second connecting assembly comprises the clamping portion; and the rotating the first connecting assembly to the clamping position, such that the first connecting assembly and the second connecting assembly are locked comprises: rotating the driving portion, until the first stop member and the second stop member are fastened, and the locking portion is rotated to the clamping portion for locking.

15. A radiotherapy system, comprising a radioactive ray generation device; and
the placement table positioning system according to any one of claims 1 to 11.
